# EUROPÄISCHE PATENTSCHRIFT

(11) **EP 1 002 080 B1**
(45) Veröffentlichungstag und Bekanntmachung des Hinweises auf die Patenterteilung: **12.03.2003**
(21) Anmeldenummer: 98940092.4
(22) Anmeldetag: 30.06.1998
(51) Int. Cl.: C12N 15/12, C12N 5/10, G01N 33/50, A01K 67/027

(54) **FLUORESZIERENDE PROTEINE ALS ZELLTYPSPEZIFISCHE REPORTER**
FLUORESCENT PROTEINS AS CELL-TYPE SPECIFIC REPORTER
PROTEINES FLUORESCENTES UTILISEES COMME RAPPORTEURS SPECIFIQUES DU TYPE CELLULAIRE

(30) Priorität: 02.07.1997 DE 19727962
(43) Veröffentlichungstag der Anmeldung: 24.05.2000
(73) Patentinhaber: AXIOGENESIS AG, 50931 Köln (DE)
(72) Erfinder: AXIOGENESIS AG, 50931 Köln (DE)
(74) Vertreter: Behnisch, Werner, Dr.
(86) Internationale Anmeldenummer: EP9803988
(87) Internationale Veröffentlichungsnummer: WO99001552

(56) Entgegenhaltungen:
- WO-A-98/36081
- ZERNICKA-GOETZ M ET AL.: "Following cell fate in the living mouse embryo " DEVELOPMENT, Bd. 124, Nr. 6, März 1997, Seiten 1133-1137, XP002086618 cambridge,uk
- WOBUS A M ET AL: "RETINOIC ACID INDUCES EXPRESSION OF THE VENTRICULAR 2.1 KB MYOSIN-LIGHT-CHAIN-2 PROMOTER DURING IN VITRO CARDIOGENESIS OF EMBRYONIC STEM CELLS" CIRCULATION, Bd. 92, Nr. 8, SUPPL. 01, 15. Oktober 1995, Seite I114 XP002001047
- LI Y ET AL: ""USE OF A GREEN FLUORESCENT PROTEIN IN STUDIES OF APOPTOSIS OF TRANSFECTED CELLLS"" BIOTECHNIQUES, Bd. 23, Nr. 6, 1997, Seiten 1026-1029, XP002068179
- WOBUS A M ET AL: "IN VITRO DIFFERENTIATION OF EMBRYONIC STEM CELLS INTO CARDIOMYOCYTES OR SKELETAL MUSCLE CELLS IS SPECIFICALLY MODULATED BY RETINOIC ACID" ROUX'S ARCHIVES OF DEVELOPMENTAL BIOLOGY, Bd. 204, Nr. 1, Oktober 1994, Seiten 36-45, XP000196431
- IKAWA M ET AL. : "Green fluorescent protein as amarker in transgenic mice" DEVELOPMENT GROWTH & DIFFERENTIATION, Bd. 37, Nr. 4, August 1995, Seiten 455-459, XP002086829 in der Anmeldung erwähnt
- ZHUO, L. ET AL: 'Live astrocytes visualized by green fluorescent protein in transgenic mice' DEV BIOL Bd. 187, Nr. 1, 01 Juli 1997, Seiten 36 - 42

## Beschreibung

Die Erfindung betrifft die Verwendung von nicht-zellschädigenden fluoreszierenden Proteinen als zelltypspezifische Reporter. Im einzelnen betrifft die Erfindung embryonale Stammzellen (ES-Zellen) nicht-menschlicher Säuger in Form von embryoid bodies, die mit einem DNA-Konstrukt stabil transfiziert sind, das eine für ein nicht-zellschädigendes fluoreszierendes Protein codierende DNA-Sequenz und einen, mit dieser DNA-Sequenz operativ verknüpften, zell- und/oder entwicklungsabhängigen Promotor umfaßt; ein Verfahren zur Herstellung dieser ES-Zellen; eine Zellkultur erhältlich durch Kultivieren der ES-Zellen; ein Verfahren zur toxikologischen Prüfung von Substanzen unter Verwendung dieser Zellkulturen; ein Verfahren zur Erzeugung transgener nicht-menschlicher Säuger unter Verwendung der ES-Zellen und ein Verfahren zur Untersuchung von Zellentwicklungsstufen unter Verwendung von Zellen, eines solchen nicht-menschlichen Säugers, wobei die ES-Zellen je ein Form von embryoid bodies vorliegen.

Embryonale Stammzellen (ES) sind Grundlage sowohl für die Erzeugung transgener Tiermodelle, als auch für den Einsatz in *in vitro* Zellkultursystem. Bisher wurden die sich aus ES-Zellen differenzierenden Zelltypen dadurch identifiziert, daß sie durch Antikörperfärbung bzw. durch in situ-Hybridisierung mit Antisense-Oligonukleotiden gekennzeichnet wurden. Dazu mußten allerdings die Zellen zuvor fixiert werden.
Alle bisherigen Verfahren eignen sich nicht für anschließende funktionelle Untersuchungen mit den von ES-Zellen differenzierten, unterschiedlichen Zelltypen. Die bisher einzige Methode, um funktionelle Untersuchungen an ES-Zell-abgeleiteten Zellderivaten *in vivo* durchzuführen, bestand in der morphologischen Identifizierung. Dies gelang, wenn auch nur sehr unbefriedigend, bei den Herz- und Skelettmuskelzellen, da diese sich durch Kontraktionen auszeichneten. Schon bei den nicht kontrahierenden Ventrikelzellen war die Erkennung für funktionelle Untersuchungen schwierig. So wird im Maltsev et al., 1994, Circ Res., 75, 233 - 244 und DD-299439 A5 ein Modell beschrieben, in dem die Differenzierung von Herzzellen (Kardiomyozyten) ausgehend von einer sehr frühen Entwicklungsstufe bis zur spezialisierten Schrittmacher-, ventrikulären-, oder atrialen Herzzelle *in vitro* stattfindet (Maltsev et al., 1994, Circ Res., 75, 233 - 244). Zu diesem Zweck werden totipotente embryonale Stammzellen (ES-Zellen) der Zellinie D3 unter den folgenden Zellkulturbedingungen in Kardiomyozyten differenziert: Die Zellen werden 2 Tage in einem hängenden Tropfen angesetzt, dann 5 Tage in Suspension gehalten und anschließend ausplatiert (Maltsev et al., 1994, Circ Res., 75, 233 - 244). Innerhalb von 1-2 Tagen nach der Platierung bilden sich spontan schlagende Areale innerhalb dieser "Embryoid Bodies" (EB's). Aus diesen Arealen können mit Hilfe enzymatischer Verdauung (Kollagenase) einzelne Kardiomyozyten dissoziiert werden, die funktionellen, molekularbiologischen und morphologischen (Immunhistochemie, Elektronenmikroskopie) Untersuchungstechniken während den verschiedenen Differenzierungsstadien zugänglich sind. Neben den Kardiomyozyten befinden sich in den so erzeugten EB's unter anderem auch neuronale Zellen, Gliazellen, hämatopoietische Zellen, Endothelzellen (frühe Kapillare), glatte Muskelzellen, Skelettmuskelzellen, Knorpeizellen, Fibroblasten und Epithelzellen.

Darüber hinaus sind in der letzten Zeit bioluminiszente Proteine wie Green Fluorescent Protein (nachfolgend GFP) beschrieben worden (Prasher et al., Gene, Vol. 111 229 - 233 (1992), die als Marker für Genexpression vorgeschlagen werden (Chalfie et al., Science, Vol. 263, 802 - 805 (1994)). So ist in der WO-A-95/07463 und WO-A-96/27675 das Transformieren von Zellen mit GFP offenbart. Die Transformation von Säugerzellen, insbesondere deren ES-Zellen mit einer für GFP codierenden DNA-Sequenz ist jedoch weder in dieser noch in einer anderen Druckschrift beschrieben worden.

Herz-Kreislauferkrankungen gehören immer noch zu den häufigsten Todesursachen in den westlichen Industrieländern. Nur durch intensive Grundlagenforschung auf diesem Gebiet können die pathophysiologischen Ursachen erkannt und neue Therapieansätze gefunden bzw. toxikologische Veränderungen beschrieben werden. Für die Untersuchung zur Pathogenese von Herz-Kreislauf Erkrankungen und zum Testen neuer pharmakologischer und toxikologischer Substanzen werden Modelle benötigt, die einerseits auf den Menschen übertragbar sind, andererseits aber die aufwendigen und kostenintensiven Tierversuchsmodelle ersetzen können. Im Jahr 1991 wurden noch über 2 Millionen Tiere allein in den alten Bundesländern bei Tierversuchen eingesetzt.
Ein in letzter Zeit immer wichtigerer Ansatzpunkt der pharmakologisch/toxikologischen Forschung ist die Herzdifferenzierung. Aus der stereotypisch ablaufenden Herzzellentwicklung können Rückschlüsse auf pathologische und toxikologische Veränderungen von Kardiomyozyten gezogen werden. So ist z.B. bekannt, daß bei der kardialen Hypertrophie (Yamazaki et al., J. Mol. Cell Cardiol. 27(1):133 - 140 (1995)) und Herzinsuffizienz (Johnson et al., Biochem. Pharmacol. 45(12):2365 - 2372 (1993)) der Rezeptorstatus und die intrazellulären Signalkaskaden gestört sind. Diese pathologisch veränderten Kardiomyozyten ähneln teilweise wieder Herzzellen früher Differenzierungsstadien.
Die zur Aufklärung der Eigenschaften von Herzzellen früher Differenzierungsstadien notwendigen Untersuchungen können am Tier jedoch technisch nur schwer und, falls überhaupt, in sehr aufwendigen Studien durchgeführt werden: Am 12.-13. Tag ist es frühestens möglich, Kardiomyozyten aus einem Mäuseembryo zu präparieren, diese Zellen entsprechen jedoch nicht mehr einer frühen kardialen Differenzierungsstufe. Eine detaillierte Analyse der Rezeptorexpression während verschiedener Differenzierungsstadien erfordert einen sehr hohen Aufwand an Tiermaterial und ist - wie vorstehend ausgeführt - technisch nur schwer durchführbar. Ebenso ist die Beobachtung der Entwicklung einer relativ undifferenzierten Herzzelle über mehrere Tage bis Wochen hinweg am Tiermodell nicht möglich. Um den Einsatz neuer Therapeutika, z.B. inotroper Substanzen oder Antiarrhythmika bzw. toxischer Stoffe, z. B. Schwermetalle oder Retinoide, auszutesten, muß im Tierversuch ein wochenlanges, invasives Monitoring an Tieren z.B. Schweinen durchgeführt werden.

Zernicka-Goetz et al., Development 124, 1133-1137, 1997 beschreiben die Herstellung einer stabilen embryonalen Stammzelllinie, die eine neue Form eines grünfluoreszierenden Proteins mit Namen MmGFP enthält und die Einführung dieser fluoreszierenden Zellen in Mäuseembryonen. Hierzu wurde MmGFP cDNA unter Kontrolle des humanen cdc2-Promotors gestellt, der in ruhenden Zellen reprimiert ist. Dieses Konstrukt wurde in ES-Zellen durch Elektroporation eingeführt. MmGFP unter Kontrolle des cdc2-Promotors wirkt als Marker in roliferierenden Zellen. Um dies nachzuweisen, wurde in MmGFPexprimierenden ES-Zellen der Zellzyklus unterbrochen und eine Differenzierung in Zellkultur induziert. In den differenzierten, sich nicht teilenden Zellen konnte dann keine MmGFP-Fluoreszenz mehr nachgewiesen werden.

Die Aufgabe der vorliegenden Erfindung bestand nun darin, ein Zellkulturverfahren bereitzustellen, das eine einfache Charakterisierung der lebenden Zellen ermöglicht und funktionelle Untersuchungen zuläßt und nicht wie die bisherigen Verfahren auf Reportergenen wie z.B. Lac-Z (Niwa et al., 1991, Gene 108, 193-199; Wobus et al., 1996, J. Cell Mol. Cardiol.; Metzger et al., 1996 Circ. Res., 78, 547-552) beruht, deren Expression nur nach Zellfixierung und unter Zuhilfenahme eines spezifischen Substrates nachgewiesen werden kann.

Überraschenderweise wurde gefunden, daß ES-Zellen mit einem DNA-Konstrukt, in dem ein für ein nicht-zellschädigendes fluoreszierendes Protein codierendes Gen mit einem zell- und entwicklungsabhängigen Promotor gekoppelt ist, mittels Elektroporation stabil transfiziert werden können. Dieses Konstrukt wird dabei in die native DNA integriert. Nach spezifischer Aktivierung intrazellulärer Signale wird der Promotor aktiviert und das fluoreszierende Protein exprimiert. Somit könnten ES-Zellen, die zu einem gewissen Zeitpunkt der Differenzierung einen zellspezifischen Transkriptionsfaktor aktivieren, anhand der Fluoreszenz-Emission unter Fluoreszenzanregung erkannt werden, wobei die ES-Zellen im Form von embryoid bodies vorliegen.

Die vorliegende Erfindung betrifft somit embryonale Stammzellen (ES-Zellen) nicht-menschlicher Säuger in Form von embryoid bodies und hegestellt nach der Methode des "hängenden Tropfens" oder durch Methylcellulosekultur, die mit einem DNA-Konstrukt, umfassend
- eine für ein nicht-zellschädigendes fluoreszierendes Protein codierende DNA-Sequenz und
- einen mit dieser DNA-Sequenz operativ verknüpften, zell- und/oder entwicklungsabhängigen Promotor
stabil transfiziert sind.
Bevorzugt stammen die ES-Zellen von Nagern, insbesondere von Mäusen. Besonders bevorzugte ES-Zellen sind dabei D3-Zellen (Doetschmann et al., J. Embryol. Exp. Morphol. 87, 27 (1985)), R1-Zellen (Nagy et al., PNAS (1995)), E14-Zellen Handyside et al., Roux Arch. Develop. Biol. 198, 48 (1989)), CCE-Zellen (Bradley et al., Nature 309, 255 (1985)) und P19-Zellen (Mummery et al., Dev. Biol 109, 402 (1985)).

Als "nicht-zellschädigendes fluoreszierendes Protein" können gemäß der vorliegenden Erfindung das Green Fluorescent Protein (GFP) aus der Qualle *Auequorea Victoria* (beschreiben in WO-A-95/07463, WO-A-96/27675 und WO-A-95/21191) und dessen Derivate "Blue GFP" (Heim et al., Curr. Biol. 6(2):178 - 182 (1996)) und "Red-shift GFP" (Muldoon et al.; Biotechniques 22(1):162 - 167 (1997)) verwendet werden. Bevorzugt ist dabei das grün fluoreszierende GFP, insbesondere die in dem hinterlegten Stamm DSM 11633 befindliche GFP-Mutante.

Unter den Begriff "zell- und/oder entwicklungsabhängiger Promotor" ist gemäß der vorliegenden Erfindung ein Promotor zu verstehen, der nur in gewissen Zelltypen und/oder nur in gewissen Zellentwicklungsstadien - sowohl in Zellkulturen (Embryoid Bodies) als auch in transgenen nicht-menschlichen Säugern, die von den erfindungsgemäßen ES-Zellen abstammen - seine Promotortätigkeit entfaltet Daneben kann aber auch jeder andere bekannte zellspezifische Promotor für z. B. Nervenzellen, Herzzellen, neuronale Zellen, Gliazellen, hämatopoietische Zellen, Endothelzellen, glatte Muskelzellen, Skelettmuskelzellen, Knorpelzellen, Fibroblasten und Epithelzellen eingesetzt werden.

In einer bevorzugten Ausführungsform der Erfindung ist der Promotor ein für Herzzellen spezifischer Promotor. Insbesondere sind die folgenden Promotoren zu nennen: SMHC Minimal Promotor (spezifisch für Glattmuskelzellen, Kallmeier et al., J. Biol. Chem. 270(52):30949 - 30957 (1995)); Nkx-2.5 (spezifisch für sehr frühe Kardiomyozyten, Lints et al., Development, 119(2):419 - 431 (1993)); Human-α-Actin (spezifisch für Herzgewebe, Sartorelli et al., Genes Dev., 4(10):1811 - 1822 (1990)), MLC-2V (spezifisch für Herzkammern, O'Brien et al., Proc. Natl. Acad. Sci. USA, 90(11):5157 - 5161 (1993) und WO-A-96/16163).

In einer bevorzugten Ausführungsform weist das DNA-Konstrukt noch weitere funktionelle DNA-Sequenzen, insbesondere Enhancer und Selektionssequenzen auf. Solche Selektionsequenzen sind z. B. Neomycin und Hygromycin.

Gegenstand der Erfindung ist weiterhin, ein Verfahren zur Herstellung der erfindungsgemäßen ES-Zellen in Form von embryoid bodies, umfassend
- Einbringen eines vorstehend definierten DNA-Konstrukts in Ausgangs-ES-Zellen nicht-menschlicher Säuger und
- Screenen nach stabil transfizierten ES-Zellen, sowie Ausbilden von embryoid bodies nach der Methode des "längenden Tropfens" oder durch Methylcellulosekultur. Das Einbringen kann dabei auf jede dem Fachmann bekannte Art und Weise erfolgen. Die Elektroporation ist jedoch bevorzugt. Das Screenen erfolgt vorzugsweise mit Hilfe der in dem DNA-Konstrukt vorhandenen Selektionssequenzen.

Gegenstand der Erfindung ist ebenfalls das vorstehend beschriebene DNA-Konstrukt. Bevorzugte Konstrukte sind dabei die Reporterkonstrukte pCX-(β-act)GFP-Neo und pCX-(α-act)GFP-Neo (DSM 11633).

Die Erfindung betrifft ebenfalls eine Zellkuttur mit zelltypspezifischer Expression eines nicht-zellschädigenden fluoreszierenden Proteins erhältlich durch Kultivieren der erfindungsgemäßen ES-Zellen. In einer bevorzugten Ausführungsform liegen die Zellen als Aggregate (Embryoid Bodies) vor. Das Herstellen des Embryoid Bodies erfolgt dabei gemäß Standardverfahren wie z. B. die Methode des "hängenden Tropfens" oder Methylzellulosekultur (Wobus et al., Differentiation (1991) 48, 172- 182).

Diese Zellstrukturen können in Verfahren zur toxikologischen Untersuchung von Substanzen, z. B. Retinoide, Schwermetall und Pharmaka, verwendet werden. Sie besitzen wesentliche Vorteile gegenüber allen bisher verwendeten Zellkulturmodellen:
a) Die lebenden, unfixierten Zellen können in ihrer Differenzierung beobachtet werden, so kann z. B. das Wachstum der Herzzellen im schlagenden Areal kontinuierlich beobachtet werden.
b) Zellen im frühen Entwicklungsstadium können elektrophysiologischen und anderen Meßmethoden zugänglich gemacht werden, da sie als fluoreszierende Zellen leicht zu erkennen sind. Dies bedeutet eine wesentliche Vereinfachung der funktionellen Untersuchung dieser Zellen.
c) Eine Einzelzell-Präparation bedeutet einen Verlust von Zellen. Um die geringe Zahl der noch vorhandenen Zellen sichtbar zu machen, sind die das fluoreszierende Protein exprimierenden Zellen vorteilhaft. Das Vefahren läßt sich durch die FACS-Sortierungsmethode ergänzen, wodurch homogene Zellpopulationen gewonnen werden können. Dadurch ist es auch möglich Untersuchungen (z.B. molekularbiologische) an einer größeren Population von phänotypisch differenzierten ES-Zellen vorzunehmen.
d) Da die das fluoreszierende Protein exprimierenden ES-Zellen nach Aktivierung herzspezifischer Promotoren im EB sichtbar werden, kann das Wachstum der herzspezifischen Zellen unter pharmakologisch/toxikologischen Bedingungen in einem recht einfachen Verfahren bestimmt werden. Für routinemäßige Untersuchungen der Wirkung verschiedener Substanzen auf die Herzzelldifferenzierung könnte das Areal im EB von fluoreszierendes Protein exprimierenden Zellen zu verschiedenen Zeitpunkten bestimmt werden und somit Aussagen gemacht werden, ob diese Substanzen die Differenzierung von Herzzellen quantitativ und qualitativ beeinflussen. Für quantitativ präzisere Aussagen könnten die Zellen dissoziiert und dann der FACS-Sortierungstechnik unterzogen werden.

Die Erfindung betrifft weiterhin ein Verfahren zur Erzeugung transgener nicht-menschlicher Blastozysten, die in der Lage sind, sich zu transgenen nicht-menschlichen Säugern mit zelltyp-spezifischer oder entwicklungsabhängiger Expression eines nicht-zellschädigenden fluoreszierenden Proteins zu entwickeln, umfassend
- Injizieren von ES-Zellen gemäß irgendeinem der Ansprüche 1 bis 7 in Blastozysten von nicht-menschlichen Säugern.

Die Erfindung betrifft weiterhin ein Verfahren zur Untersuchung von Entwicklungsstufen von Zellen nicht-menschlicher Säuger mit den Schritten:
- Erzeugen von transgenen nicht-menschlichen Blastozysten, die in der Lage sind, sich zu transgenen nicht-menschlichen Säugem mit zelltyp-spezifischer oder entwicklungsabhängiger Expression eines nicht-zellschädigenden fluoreszierenden Proteins zu entwickeln, nach dem Verfahren nach Anspruch 12;
- Untersuchen der das fluoreszierende Protein exprimierenden Zellen durch fluorimetrische Verfahren in vitro.

Im Ganztier kann durch das erfindungsgemäße Untersuchungsverfahren erstmals *in vivo* eine genaue Zelltypisierung vorgenommen werden. So sollten bereits in den frühen Organanlagen der Embryonen fluoreszierende Herzzellen beobachtet werden, was eine *in vivo* Beobachtung der Herzentwicklung in der frühembryonalen Phase möglich machen würde. Auch dadurch könnten zu verschiedenen Entwicklungsstufen die Herzzellen leicht identifiziert werden.

Die Erfindung wird weiterhin durch die folgende Beispiele erläutert.

Das Beispiel 1 verwendet Reportergenkonstrukt pCX-(β-act)GFP-Neo.
Das Beispiel 2 verwendet Reportergenkonstrukt pCX-(α-act)GFP-Neo (DSM 11633).
In jungen EBs in Suspension unter transmittiertem Licht (Aa) und unter 488 nm Exzitation (Ab) ist keine grüne Fluoreszenz sichtbar da sich in diesen frühen EBs noch keine Herzzellen differenziert haben.
EB nach 3 Tagen Platierung (7+3 Tage) unter transmittiertem Licht (Ba), bei 488 nm Fluoreszenzexzitation (Bb) und in Kombination von transmittiertem Licht und 488 nm Fluoreszenzexzitation. In diesem EB konnte ein relativ kleines, spontan schlagendes Areal beobachtet werden. Dies entspricht dem Teil des EB mit spontanen Kontraktionen.

Zum Beispiel 2d, Mittels FACS-Methode werden Verteilungshistogramme von Zellen aus EB's, die aus ES-Zellen mit pCX-(α-act)GFP-Neo gewonnen wurden, erstellt. Das Konstrukt wurde mittels Aatl linearisiert, so daß der CMV-IE Enhancer zerstört wurde.
(a) ES-Zellen
(b) nach 2 Tagen Suspension (2+2 Tage)
(c) 5 Tage nach Platieren (7+5 Tage)
x-Achse: Intensität der GFP Fluoreszenz (FACS-Einheiten). y-Achse: gezählte Zellen

### Beispiele

### Beispiel 1: Herstellung stabiler ES-Zellinien, die GFP unter einem starken ubiquitären Promotor exprimieren, und Differenzierung und Eigenschaften von Kardiomyozyten, die von diesen Zellen abstammen.

a) Herstellen des GFP-Expressions-Konstrukts: Der von Dr. Okabe (University of Osaka, Japan) zur Verfügung gestellte pCX-GFP-Expressionsvektor, der eine GFPcodierende Sequenz unter einem Huhn-α-Actin-Promotor enthält (M. Ikawa, K. Kominami, Y. Yoshimura, K. Tanaka, Y. Nishimune und M. Okabe, Develop. Growth Differ. (1995) 37, 455-459), wurde wie folgt modifiziert:
   Ein Sall-Xbal-Restriktionsfragment, das ein Neomycin-(G418)-Resistenz-Gen von pTL2Neo enthielt (von Dr. Tarakhovsky zur Verfügung gestellt), wurde durch Ligasierung mit glatten Enden in die Sall-Stelle von pCX-GFP eingesetzt. Das resultierende Konstrukt pCX-(β-act)GFP-Neo wurde für die Elektroporation von D3-Zellen verwendet.
b) Elektroporation und Selektionsverfahren: Das pCX-(β-act)GFP-Neokonstrukt wurde mit Restriktase Scal linearisiert (außerhalb der GFP-Expressionscassette) und für die Elektroporation der D3-Linie von ES-Zellen unter den folgenden Bedingungen verwendet:
   DNA: 20-40 µg, Zellen: 7 x 10/ml in 0,8 ml PBS-Puffer, Elektroporationsküvette BioRad 0,4 cm (Kat.-Nr. 165-2088), Elektroporationsapparatur BioRad (Gen Pulser), 240 V, 500 µF.
   Nach der Elektroporation wurde die Zellsuspension 20 min auf Eis gelegt und dann auf eine 10-cm-Petrischale mit Gewebequalität mit G418-Resistenz-Feederschicht in 10 ml DMEM-Medium mit 15% FCS (Kälberfetusserum) übertragen. Zwei Tage später wurde 300 µg/ml Neomycin (G418. Gibeo) hinzugefügt, um G418-resistente Zellen zu selektieren. Das Medium mit G418 (300 µg/ml) wurde jeden zweiten Tag ausgetauscht. Nach 8 bis 10 Tagen Selektion erschienen wirkstoffresistente Kolonien, die durch Fluoreszenzmikroskopie auf GFP-Expression getestet wurden.
   Etwa 95% der G418-resistenten Kolonien zeigten eine starke GFP-Expression (grünes Leuchten), was auf ein hohes Ausmaß an Aktivität des β-Actin-Promotors (β-Actin ist eines der Hauptproteine des Cytoskeletts) in ES-Zellen hinweist. Die Kolonien wurden durch Ansaugen mit einer Pasteur-Pipette aufgenommen, einzeln trypsinisiert und anschließend für die Vermehrung auf 48- und 24-Napf-Platten mit Feederschichten mit G418 (300 µg/ml) übertragen. Schließlich wurden mehrere stabile ES-Klone, die 1 bis 5 Kopien des GFP-Gens unter der Kontrolle des Huhn-β-Actin-Promotors trugen, ermittelt und zur Erzeugung embryoider Körper (EBs) verwendet.
c) Differenzierung und Analyse der Kardiomyozyten: EBs wurden nach dem Standardverfahren der "hängenden Tropfen" entwickelt (A. Wobus, G. Walluka und J. Hescheler; Differentiation (1991) 48, 173-182).
   In allen Stadien der Entwicklung vor dem Ausstreichen zeigten die EBs, die von ES-Zellen mit integriertem GFP-Expressionsvektor unter der Kontrolle des β-Actin-Promotors abstammten, unter dem Fluoreszenzmikroskop ein starkes grünes Leuchten. Nach dem Ausstreichen verteilte sich die GFP-Expression in ungleichen Anteilen zwischen verschiedenen Zelltypen, die im Laufe der Differenzierung auftraten. Das hellste grüne Leuchten in EBs nach dem Auftreten kontraktiler Myocardiocyten fällt mit entsprechenden schlagenden Bereichen und nicht mit schlagenden Kernteilen (zentralen Teilen) von EBs zusammen.
   Andere Bereiche der EBs zeigen verschiedene Ausmaße der GFP-Expression von stark bis schwach, was auf die bekannte weit verbreitete Expression von β-Actin als Hauptkomponente des Cytoskeletts bei vielfältigen Zelltypen hinweist.
   Diese visuellen Beobachtungen werden durch Histogramme der Verteilung der GFP-Expression in den Zellpopulationen in den sich entwicklenden EBs bestätigt, die man durch FACS-Analyse (Flow Cytofluorimetry) erhält. Sie zeigen die Linksverschiebung und Vergrößerung anfangs scharfer, symmetrischer Peaks des Histogramms, was einen Übergang von hoher und relativ homogener GFP-Expression in einer Population proliferierender undifferenzierter ES-Zellen zu einer breiten Verteilung derselben auf die Population sich differenzierender Zelltypen anzeigt.
   EBs wurden mittels Kollagenase dissoziiert und auf Objektträgern ausgestrichen und anschließend 2-3 Tage lang kultiviert, bevor elektrophysiologische Messungen nach dem Standardverfahren vorgenommen wurden (V. Maltsev, A.Wobus, J. Rohwedei, M. Bader und J. Hescheler; Circ. Res. (1994) 75(2), 233-244).
   Isolierte, spontan schlagende Kardiomyozyten, die GFP stark exprimieren, weisen alle elektrophysiologischen Eigenschaften auf, die für Kardiomyozyten typisch sind einschließlich Aktionspotentialen, Ca²⁺-, Na⁺-, K⁺- und Iᵣ-Strömen, was durch die Patch-Clamp-Technik gezeigt wurde.
   Stabile ES-Zellinien, die aufgrund eines in das Genom integrierten Expressionsvektors GFP stark exprimieren, können sich also zu funktionell reifen kontraktilen Kardiomyozyten differenzieren, die dieselben elektrophysiologischen Grundeigenschaften haben wie Kardiomyozyten, die sich aus "normalen" D3-Zellen differenziert haben.

### Beispiel 2: Herstellung stabiler ES-Zellinien, die GFP unter einem herzspezifischen Promotor in sich differenzierenden Kardiomyozyten exprimieren.

a) Herstellen des GFP-Expressions-Konstrukts: Aus dem von Dr. M. McBumey (University of Ottawa, Kanada) zur Verfügung gestelltem Plasmid pPv/B-Act-lacZ, das das Segment (-440 +6) des herzspezifischen Human-a-Actin-Promotors enthielt (A.Minty, L. Kedes; Molecular and Cellular Biology (1986) 6, 2125-2136; G. Pari, K. Jardine und M. McBumey; Molecular and Cellular Biology (1991) 11, 4796-4803), wurde der Promotor durch die Restriktionsenzyme SaII und HindIII herausgeschnitten. Der pCX-GFP-Expressionsvektor (siehe Beispiel 1) wurde - um den Huhn-β-Actin-Promotor durch den herzspezifischen α-Actin-Promotor zu ersetzen - mit den Restriktasen SnaBI und Apal gespalten, wobei der Huhn-β-Actin-Promotor herausgeschnitten wurde. Nachfolgend wurde durch Ligasierung mit glatten Enden das oben genannte SaII-I-HindIII-Fragment des herzspezifischen α-Actin-Promotors eingefügt. Die Restriktasen Tth111I, Sall und HinfI wurden verwendet, um Plasmidklone zu selektieren, bei denen der herzspezifische α-Actin-Promotor in der richtigen Orientierung zur GFP-codierenden Sequenz eingesetzt war. Dann wurde ein Neomycin-(G418)-Resistenz-Gen, wie in Beispiel 1a) beschrieben, in die Sall-Stelle eingesetzt, und das resultierende Plasmid pCX-(α-act)GFP-Neo wurde für die Elektroporation von D3-Zellen verwendet.
b) Elektroporation und Selektionsverfahren: Das pCX-(α-act)GFP-Neo wurde mit Scal (außerhalb der GFP-Expressionscassette, wie in Beispiel 1b) beschrieben) oder mit Aatl linearisiert, um den Cytomegalovirus-(CMV-IE)-Enhancer zu zerstören (siehe unten). Die Elektroporation und das G418-Selektionsverfahren wurden, wie in Beispiel 1b) beschrieben, durchgeführt.
c) Analyse der Zellpopulation und GFP-Expression unter der Kontrolle des herzspezifischen α-Actin-Promotors während der Differenzierung von Kardiomyozyten: EBs wurden, wie in Beispiel 1c) beschrieben, entwickelt. Im Unterschied zu den oben beschriebenen Mustern der GFP-Expression unter der Kontrolle des β-Actin-Promotors weisen ES-Zellen, die das in das Genom integrierte pCX-(α-act)GFP-Neo tragen, kein oder nur ein sehr schwaches durch Fluoreszenzmikroskopie sichtbares Signal auf. Die FACS-Analyse zeigt, daß das mittlere Niveau der grünen Fluoreszenz von D3-Zelle mit durch Sacl linearisiertem pCX-(α-act)GFP-Neo etwa 35- bis 40mal geringer ist als bei Zellinien, die pCX-(β-act)GFP-Neo tragen. Während der Entwicklung der EBs wurde eine Rechtsverschiebung und Vergrößerung des Anfangspeaks des durch FACS erhaltenen GFP-Fluoreszenz-Histogramms erhalten. Die schlagenden Bereiche, die 2 bis 4 Tage nach dem Ausstreichen der EBs auftraten, zeigen ein durch Fluoreszenzmikroskopie sichtbares grünes Leuchten, das mit dem Niveau der GFP-Expression in ES-Zellen, die pCX-(β-act)GFP-Neo tragen, vergleichbar ist. Möglicherweise sind die schlagenden Bereiche, die aus funktionell reifen Kardiomyozyten bestehen, nur Bereiche mit starkem sichtbarem Leuchten unter anderen Zelltypen in sich entwickelnden EBs. Bei täglicher Überwachung ist es möglich, 1 bis 1,5 Tage, bevor sie zu schlagen beginnen, getrennte leuchtende Bereiche nachzuweisen.
   Der herzspezifische Charakter der GFP-Expression wurde durch α-Actin-spezifisches Immun-Anfärben einzelner Zellen bestätigt, das mit der GFP-Expression unter der Kontrolle des herzspezifischen α-Actin-Promotors korreliert.
d) Herstellung von ES-Zellinien mit geringem ursprünglichem unspezifischem Niveau der GFP-Expression: Die oben genannten ES-Klone mit integriertem pCX-(α-act)GFP-Neo zeigten eindeutige Kardiomyozyten-spezifische Expressionsmuster, was zu einem erkennbaren Zusammenfallen leuchtender und schlagender Bereiche führte. Das anfängliche Niveau der GFP-Expression dieser undifferenzierten Zellen, das 5- bis 10mal höher liegt als bei einer negativen Kontrolle (intakte ES-Zellen), könnte jedoch das Auffinden der Zellen bei den allerersten Schritten der Differenzierung behindern.

Um das ursprüngliche Niveau der unspezifischen GFP-Expression zu senken, wurde eine Anzahl neuer Zellinien erzeugt, wobei pCX-(α-act)GFP-Neo verwendet wurde, das mit Aatl-Restriktase linearisiert wurde, um eine Zerstörung des CMV-IE-Enhancers zu ermöglichen Letzterer war im ursprünglichen Expressionsvektor vorhanden, und es wurde angenommen, daß er der Grund für den anfänglichen unspezifischen "Hintergrund" war.

Nach der Elektroporation und dem Selektionsverfahren zeigten die G418-resistenten Klone eine viel höhere Diversifikation der anfänglichen GFP-Expression als Klone mit intaktem CMV-IE-Enhancer. Mehrere ES-Klone mit niedrigstem "Hintergrund" wurden durch FACS-Screening ausgewählt. Diese Klone haben eine etwa 5mal niedrigere Anfangsfluoreszenzintensität und könnten mit Zellen der negativen Kontrolle (Wildtyp) ohne GFP-Vektor vergleichbar sein. Nach der Herstellung von EBs aus entsprechenden ES-Zellklonen zeigten mehrere davon eine sowohl durch Fluoreszenzmikroskopie als auch durch FACS-Analyse nachweisbare GFP-Expression in sich differenzierenden Kardiomyozyten. Die FACS-Analyse zeigt eine hohe Auflösung zwischen Zellen, die zur herzspezifischen Differenzierung übergegangen sind, und dem Rest der Zellpopulation, die während der EB-Entwicklung den ausgeprägten eins-zu-zwei-Peakcharakter der Histogrammdynamik zeigten.

Der beschriebene Ansatz erlaubt es also, die Differenzierung von ES-Zellen zu Kardiomyozyten "in vitro" zu untersuchen, wobei der GFP-Expressionsvektor mit herzspezifischem Promotor als "lebendes" Repoter-Gensystem verwendet wird, der Zellen in den früheren Schritten der Entwicklung sichtbar macht.

Das vorstehend beschriebene Plasmid pCX-(α-act)GFP-Neo wurden am 27.06.97 bei der Deutschen Sammlung von Mikroorganismen und Zellkulturen GmbH, Maschroder Weg 1b, D-38124 Braunschweig, unter der Bezeichnung DSM 11633 hinterlegt.

## Patentansprüche

1. Zellkulturen mit zelltypspezifischer oder entwicklungsabhängigen Expression eines nicht-zellschädigenden fluoreszierenden Proteins aufgrund einer Aktivierung eines zellspezifischen oder entwicklungsablängigen, Transkriptionsfaktors zu einem gewissen Zeitpunke der Differenzierung, bestehend aus Aggregaten (Embryoid Bodies) embryonaler Stammzellen (ES-Zellen) nicht-menschlicher Säuger hergestellt durch die Methode des "hängenden Tropfens" oder durch Methylcellulosekultur, die mit einem DNA-Konstrukt, umfassend
- eine für das nicht-zellschädigendes fluoreszierendes Protein codierende DNA-Sequenz und
- einen mit dieser DNA-Sequenz operativ verknüpften, zeit- und/oder entwicklungsabhängigen Promotor stabil transfiziert sind, wobei das DNA-Konstrukt in die native DNA integriert ist.

2. Zellkulturen gemäß Anspruch 1, wobei die ES-Zellen von Nagern, insbesondere von Mäusen, stammen.

3. Zellkulturen gemäß Anspruch 1 oder 2, wobei das nicht-zellschädigende fluoreszierende Protein ausgewählt ist aus Green Fluorescent Protein (GFP), Red Fluorescent Protein und Blue Fluorescent Protein.

4. Zellkulturen gemäß irgendeinem der Ansprüche 1 bis 3, wobei der Promotor ein für Herzzellen, neuronale Zellen, Gliazellen, hämatopoietische Zellen, Endothelzellen, glatte Muskelzellen, Skelettmuskelzellen, Knorpelzellen, Fibroblasten oder Epithelzellen spezifischer Promotor ist.

5. Zellkulturen gemäß Anspruch 4, wobei der Promotor ausgewählt ist aus den Promotoren Nkx-2.5, Human-α-Actin und MLC-2V und insbesondere der herzspezifische Human-α-Actin Promotor ist.

6. Zellkulturen gemäß irgendeinem der Ansprüche 1 bis 5, wobei das DNA-Konstrukt weitere funktionelle DNA-Sequenzen, insbesondere Enhancer- und Selektionssequenzen, aufweist.

7. Zellkulturen gemäß Anspruch 1, wobei das DNA-Konstrukt das Plasmid pCX-(α-act)GFP-Neo (DSM 11633) ist.

8. Verfahren zur Herstellung der Zellkulturen gemäß irgendeinem der Ansprüche 1 bis 7, umfassend
- Einbringen eines wie in Anspruch 1 und 3 bis 7 definierten DNA-Konstrukts in Ausgangs-ES-Zellen nicht-menschlicher Säuger und
- Screenen nach stabil transfizierten ES-Zellen;
- Ausbilden von Embryoid Bodies durch die Methode des "hängenden Tropfens" oder durch Methylcellulosekultur.

9. Verfahren nach Anspruch 8, wobei das Einbringen durch Elektroporation erfolgt.

10. Verfahren gemäß Anspruch 8 oder 9, weiterhin umfassend Kultivieren der stabil transfizierten; ES-Zellen in Form von embroyoid bodies *in vitro*.

11. Verfahren zur toxikologischen Prüfung von Substanzen, umfassend die Untersuchung der Auswirkungen dieser Substanzen auf die Zellkulturen gemäß Ansprüchen 1 bis 7 mittels fluorimetrischer Verfahren.

12. Verfahren zur Erzeugung transgener nicht-menschlicher Blastozysten, die in der Lage sind, sich zu transgenen nicht-menschlichen Säugern mit zelltyp-spezifischer oder entwicklungsabhängiger Expression eines nicht-zellschädigenden fluoreszierenden Proteins zu entwickeln, umfassend
- Injizieren von ES-Zellen gemäß Irgendeinem der Ansprüche 1 bis 7 in Blastozysten von nicht-menschlichen Säugern.

13. Verfahren zur Untersuchung von Entwicklungsstufen von Zellen nicht-menschlicher Säuger mit den Schritten:
- Erzeugen von transgenen nicht-menschlichen Blastozysten, die in der Lage sind, sich zu transgenen nicht-menschlichen Säugern mit zelltyp-spezifischer oder entwicklungsabhängiger Expression eines nicht-zellschädigenden fluoreszierenden Proteins zu entwickeln, nach dem Verfahren nach Anspruch 12;
- Untersuchen der das fluoreszierende Protein exprimierenden Zellen durch fluorimetrische Verfahren in vitro.

## Claims

1. Cell cultures exibiting cell-type specific or development-specific expression of an non-cell-damaging fluorescent protein due to activation of a cell-specific or development-specific transcription factor at a certain point of differentiation, consisting of aggregates (embryoid bodies) of embryonic stem cells (ES cells) of non-human mammals obtained by the "hanging drop" method or by methylcellulose culture, stably transfected with a DNA construct comprising:
- a DNA sequence coding for said non-cell damaging fluorescent protein; and
- a cell-dependent or development-dependent promoter operably linked with said DNA sequence,
said DNA construct being integrated in the native DNA.

2. Cell cultures according to claim 1, wherein said ES cells are derived from rodents, especially mice.

3. Cell cultures according to claim 1 or 2, wherein said non-cell-damaging fluorescent protein is selected from Green Flourescent Protein (GFP), Red Fluorescent Protein and Blue Fluorescent Protein.

4. Cell cultures according to any of claims 1 to 3, wherein said promoter is a promoter specific for heart cells, neurons, glia cells, hematopoietic cells, endothelial cells, smooth muscle cells, skeletal muscle cells, cartilage cells, fibroblasts or epithelial cells.

5. Cell cultures according to claim 4, wherein said promoter is selected from Nkx-2.5, human α-actin and MLC-2V promoters, especially being the heart specific human α-actin promoter.

6. Cell cultures according to any of claims 1 to 5, wherein said DNA construct includes further functional DNA sequences, especially enhancer and selective sequences.

7. Cell cultures according to claim 1, wherein said DNA construct is plasmid pCX-(α-act)GFP-Neo (DSM 11633).

8. A method for preparing the cell cultures according to any of claims 1 to 7, comprising:
- introducing a DNA construct as defined in claims 1 and 3 to 7 in starting ES cells on non-human mammals; and
- screening for stably transfected ES cells;
- forming of embryoid bodies by the "hanging drop" method or by methylcellulose culture.

9. The method according to claim 8, wherein said introducing is effected by electroporation.

10. Method according to claim 8 or 9, further comprising culturing said stably transfected ES cells in form of embryoid bodies in vitro.

11. A method for the toxicological examination of substances, comprising the examination of the effects of said substances on the cell cultures according to claims 1 to 7 using fluorimetric methods.

12. A method for producing transgenic non-human blastocysts which are able to develop into transgenic non-human mammals with cell-specific or development-specific expression of a non-cell-damaging fluorescent protein, comprising:
- injecting ES cells according to any one of claims 1 to 7 into blastocysts of non-human mammals.

13. A method for examining stages of development of non-human mammal cells comprising the following steps:
- producing transgenic non-human blastocysts able to develop into transgenic non-human mammals with cell-specific or development-specific expression of a non-cell-damaging fluorescent protein according to the method of claim 12;
- examining said cells expressing said fluorescent protein by fluorimetric methods in vitro.

## Revendications

1. Cultures cellulaires à expression spécifique de type cellulaire ou dépendante du développement, d'une protéine fluorescente n'endommageant pas les cellules, en raison d'une activation d'un facteur de transcription, spécifique de type cellulaire ou dépendant du développement, à un certain moment de la différenciation, constituées d'agrégats [*embryoid bodies* (= corps embryoïdes)] de cellules souches embryonnaires (cellules ES) de mammifères non humains, produites par la méthode de la "goutte suspendue" ou par culture sur méthylcellulose, qui sont transfectées de façon stable avec un produit de construction d'ADN comprenant
- une séquence d'ADN codant pour la protéine fluorescente n'endommageant pas les cellules et
- un promoteur dépendant du type de cellule et/ou du développement, fonctionnellement lié à cette séquence d'ADN,
le produit de construction d'ADN étant intégré dans l'ADN natif.

2. Cultures cellulaires selon la revendication 1, dans lesquelles les cellules ES proviennent de rongeurs, en particulier de souris.

3. Cultures cellulaires selon la revendication 1 ou 2, dans lesquelles la protéine fluorescente n'endommageant pas les cellules est choisie parmi la protéine fluorescente verte (GFP), la protéine fluorescente rouge et la protéine fluorescente bleue.

4. Cultures cellulaires selon l'une quelconque des revendications 1 à 3, dans lesquelles le promoteur est un promoteur spécifique de cellules cardiaques, de cellules neuronales, de cellules gliales, de cellules hématopoïétiques, de cellules endothéliales, de cellules musculaires lisses, de cellules de muscles squelettiques, de cellules cartilagineuses, de fibroblastes ou de cellules épithéliales.

5. Cultures cellulaires selon la revendication 4, dans lesquelles le promoteur est choisi parmi les promoteurs Nkx-2.5, d'actine α humaine et MLC-2V, et en particulier est le promoteur d'actine α humaine spécifique du coeur.

6. Cultures cellulaires selon l'une quelconque des revendications 1 à 5, dans lesquelles le produit de construction d'ADN comporte d'autres séquences d'ADN fonctionnelles, en particulier des séquences enhancer et de sélection.

7. Cultures cellulaires selon la revendication 1, dans lesquelles le produit de construction d'ADN est le plasmide pCX(-α-act)GFP-Neo (DSM 11633).

8. Procédé pour la production des cultures cellulaires selon l'une quelconque des revendications 1 à 7, comprenant
- l'introduction d'un produit de construction d'ADN défini dans les revendications 1 et 3 à 7 dans des cellules ES de mammifères non humains de départ et
- le criblage à la recherche de cellules ES transfectées de façon stable ;
- la formation d'*embryoid bodies* par la méthode de la "goutte suspendue" ou par culture sur méthylcellulose.

9. Procédé selon la revendication 8, dans lequel l'introduction s'effectue par électroporation.

10. Procédé selon la revendication 8 ou 9, comprenant en outre la culture in vitro des cellules ES transfectées de façon stable, sous forme d'*embryoid bodies.*

11. Procédé pour le contrôle toxicologique de substances, comprenant l'étude des effets de ces substances sur les cultures cellulaires selon les revendications 1 à 7, au moyen de procédés fluorimétriques

12. Procédé pour la production de blastocystes transgéniques non humains, qui sont capables de se développer en mammifères transgéniques non humains à expression spécifique de type de cellule ou dépendante du développement, d'une protéine fluorescente n'endommageant pas les cellules, comprenant
- l'injection de cellules ES selon l'une quelconque des revendications 1 à 7, dans des blastocystes de mammifères non humains.

13. Procédé pour l'étude de stades du développement de cellules de mammifères non humains, comportant les étapes suivantes :
- production de blastocystes transgéniques non humains qui sont capables de se développer en mammifères transgéniques non humains à expression spécifique de type de cellule ou dépendante du développement, d'une protéine fluorescente n'endommageant pas les cellules, conformément au procédé selon la revendication 12;
- étude des cellules exprimant la protéine fluorescente par des procédés fluorimétriques in vitro.
